# EUROPEAN PATENT APPLICATION

(11) **EP 4 173 715 A1**
(43) Date of publication of application: **03.05.2023**
(21) Application number: 22199518.6
(22) Date of filing: 04.10.2022
(51) Int. Cl.: B01L 3/00, B01L 7/00, C12Q 1/68

(54) **CHIP FOR BIOLOGICAL ANALYSIS, SYSTEM FOR BIOLOGICAL ANALYSIS, AND MANUFACTURING METHOD OF THE CHIP**

(30) Priority: 27.10.2021 IT 202100027521
(71) Applicant: STMicroelectronics S.r.l., 20864 Agrate Brianza (MB) (IT)
(72) Inventor: RAIA, Lillo, 20832 DESIO (MB) (IT); FREGUGLIA, Alessandro, 20151 MILANO (IT); PESATURO, Massimiliano, 24060 TORRE DE' ROVERI (BG) (IT)
(74) Representative: Studio Torta S.p.A.

(57) **Abstract**

A chip (1) for biochemical reactions comprising: a first body (3) including a plurality of first through openings (5) arranged according to an arrangement pattern; a second body (4), having a hydrophilic surface (8), coupled to the first body (3) on the hydrophilic surface (8); and an intermediate layer (10), which extends over the hydrophilic surface (8) and forms a coupling interface between the first and the second bodies. The intermediate layer (10) is of hydrophobic material, extends continuously over the hydrophilic surface, and has a plurality of second through openings (12) through which respective regions (8') of the hydrophilic surface (8) are exposed. The hydrophilic regions (8') may be functionalized for carrying out a PCR.

## Description

The present invention relates to a chip for biological analysis, to a system for biological analysis including this chip, and to a manufacturing method of the chip.

As is known, analysis of nucleic acids requires, according to various recognition modalities, preliminary steps of preparation of a sample of biological material (in particular, nucleic acids), amplification of the nucleic material contained therein, and hybridization of individual target or reference strands, corresponding to the sequences sought.

At the end of the preparatory steps, the sample must be examined to check whether the amplification has taken place regularly.

According to the methodology referred to as real-time PCR (Polymerase Chain Reaction), the nucleic acid is amplified through appropriately selected thermal cycles, and evolution of the amplification is detected and monitored by fluorescence during the entire process.

The amplification reactions are conducted so that the chains of nucleic acids, contained in a recognition chamber provided in a support, include fluorescent molecules or fluorophores.

Chips are known provided with recognition chambers having a hydrophilic base portion and a hydrophobic lateraledge portion. This need is achieved, according to the state of the art, by arranging on a chip with hydrophilic surface a containment structure of hydrophobic material, which defines a plurality of chambers.

In the absence of a hydrophobic confinement, the solution contained in the chambers (comprising the biological sample and the reagents) may assume an excessively peripheral distribution, both at the expense of the conditions of reaction, the uniformity of which may be compromised up to the point where the reaction itself is considerably slowed down or even prevented, and at the expense of the external detection of the signal indicative of the result of the reaction.

The aim of the present invention is to provide a chip for biological analysis, a system for biological analysis that includes this chip, and a manufacturing method of the chip that are alternative to those of the state of the art, which will enable simplification of the manufacturing method and likewise reduction of the costs of the intermediate and final products.

According to the present invention, a chip for biological analysis, a system for biological analysis that includes this chip, and a manufacturing method of the chip are provided, as defined in the annexed claims.

For a better understanding of the invention, some embodiments thereof will now be described, purely by way of non-limiting example and with reference to the attached drawings, wherein:
- Figure 1A is an exploded perspective view of a structure including a microreactor, or chip, for biochemical analysis, according to one embodiment of the present invention;
- Figure 1B is a perspective view of the structure including the microreactor of Figure 1A;
- Figures 2A and 2B show, in cross-sectional view, drops of a liquid that are arranged on a hydrophilic surface and on a hydrophobic surface, respectively;
- Figures 3-6 illustrate, in lateral sectional view, steps for manufacturing the microreactor of Figures 1A, 1B, according to the present invention;
- Figure 7 is a lateral sectional view of a detail of a single reaction chamber of the microreactor of Figures 1A, 1B, manufactured according to what is described with reference to Figures 3-6; and
- Figure 8 illustrates a system for biological analysis including the microreactor of Figures 1A, 1B.

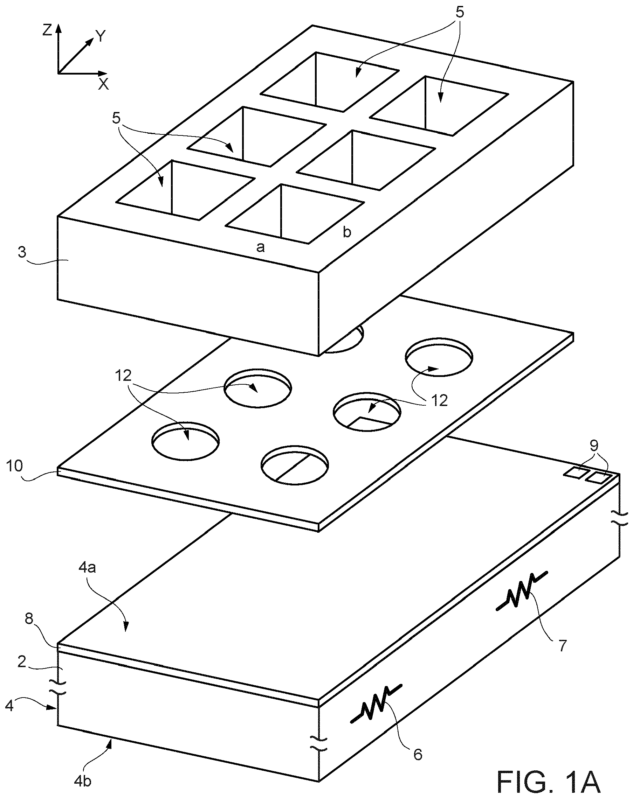

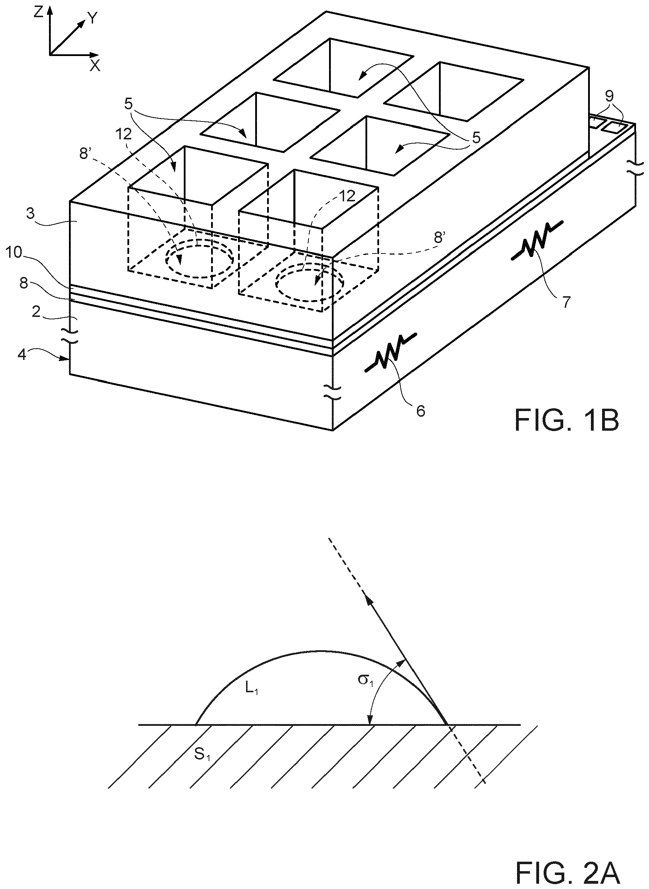

With joint reference to Figures 1A and 1B, a portion of a chip 1 for biochemical analysis (also known as microreactor) is illustrated as a whole. The chip 1 comprises a first die 3, for example of polymeric material (e.g., plastic material, typically polypropylene or polycarbonate), and a second die 4, for example including a substrate 2 of semiconductor material, such as silicon. Other materials are in any case possible according to the need.

A hydrophilic layer 8, for example of silicon oxide (SiO₂), extends over a front side 4a of the second die 4 (in this example, coinciding with a top surface of the substrate 2) .

A coupling and surface-modification layer 10, with characteristics of low wettability, namely, a hydrophobic layer, extends between the first die 3 and the second die 4 on the hydrophilic layer 8. This layer 10 has the dual function of enabling physical coupling (pasting) between the first die 3 and the second die 4 and of modifying, selectively and locally, as illustrated more fully hereinafter, the characteristics of wettability of the hydrophilic layer 8.

Hereinafter, for simplicity, the coupling and surface-modification layer 10 will be referred to as the hydrophobic layer 10.

The hydrophobic layer 10 extends continuously over the hydrophilic layer 8 and has a plurality of through openings 12, through which respective regions 8' of the hydrophilic layer 8 are exposed, or rendered accessible.

In use, a liquid solution containing biological samples to be analysed is deposited in the regions 8'. The shape, in top view (namely, in the plane XY), of the through openings 12 is defined according to the need and is, for example, a circular shape. The diameter of the through openings is selected in the range 0.2-3.0 mm.

In an embodiment, the through openings 12 are arranged, in a view in the plane XY, according to a matrix pattern (in Figure 1, in particular, a 3x2 matrix). Other patterns of arrangement are in any case possible.

The first die 3 comprises a plurality of wells 5 (in particular, equal in number to the number of through openings 12), each delimited by an inner wall 5a; the wells 5 are each designed to receive the solution containing biological samples to be analysed. The wells 5 have, in top view, a generically polygonal shape, for example quadrangular, with sides a and b, measured along axes X and Y, having a length freely chosen in the range 3 to 6 mm. The depth c of the wells 5, measured along an axis Z orthogonal to the plane defined by the axes X and Y, is chosen in the range 2 to 4 mm. The depth c of the wells 5 is equal to the thickness of the first die 3.

Alternatively, the wells 5 may have a circular or elliptical shape, respectively with a diameter or axes of dimensions comparable to those of the sides a and b and a similar depth.

The wells 5 are arranged, in a view in the plane XY, according to the same matrix pattern described for the through openings 12 and are separated from one another, along the directions X and Y, by an amount chosen according to the need, in particular to guarantee absence of cross contamination between adjacent wells (above all during the steps of introduction of the solution to be analysed). It is, however, clear that the pattern of arrangement of the wells 5 may be different from the one illustrated, and may be chosen freely, according to the need.

The shape and arrangement of the wells 5 is such that, when the first die 3 is coupled to the hydrophobic layer 10 and to the second die 4, the through openings 12 are accessible through the wells 5 to receive the solution to be analysed. In particular, the through openings 12, in a view in the plane XY, are completely contained within the wells 5.

In particular, in a view in the plane XY, the diameter of the through openings 12 is smaller than the diameter of the wells 5, so that the shape of the drop of solution to be analysed is defined by the shape and size of the through openings 12 and not by the shape and size of the wells 5. In other words, when the solution to be analysed is introduced into the wells 5, the difference between the characteristics of wettability of the regions 8' exposed through the through openings 12 and the characteristics of wettability of the hydrophobic layer 10 causes the solution that is to be analysed to be confined within the through openings 12.

Each well 5, together with the region 8' and with the portion of hydrophobic layer 10 exposed through the respective well 5, defines a PCR chamber.

According to an embodiment, heaters 6 and temperature sensors 7 are also integrated in the second die 4. The temperature sensors 7 are, for example, of a thermoresistive type (other types of sensors are, however, possible). In practice, their resistance varies as a function of the temperature, and therefore a reading of the resistance is indicative of the temperature at a given instant. The heaters 6 and the temperature sensors 7 are, for example, formed on a back side 4b, opposite to the front side 4a, of the second die 4. In this example, the back side 4b coincides with a bottom surface of the substrate 2.

In an embodiment provided by way of non-limiting example of the present invention, the second die 4 projects slightly on one side with respect to the first die 3 and on the projecting side houses contact pads 9, to provide electrical connection regions of the heaters 6 and of the temperature sensors 7 with a control-and-read card (not shown).

The heaters 6 and the temperature sensors 7 are controlled in order to implement the known amplification steps for carrying out PCR, in a per se known manner and not forming the subject of the present invention.

It is clear that various embodiments may envisage that either the heaters 6 or the temperature sensors 7 or both are of a non-integrated type, namely, coupled to the chip 1 in some other way.

According to the present invention, the hydrophobic layer 10 functions both as a containing layer for the solution to be analysed, as already mentioned, and as a coupling layer between the first and the second dice 3, 4. For this purpose, the hydrophobic layer 10 is obtained using a paste, in particular a silicone-based paste, which offers, at the same time, characteristics of hydrophobicity and compatibility with the biological material to be analysed.

Examples of materials that may be used for the hydrophobic layer 10 include: silicones, acrylates, epoxy pastes, appropriately functionalized silanes or Teflon.

In the context of the present invention, the term "hydrophobic" refers to a surface having reduced wettability, namely, such that the surface interaction between a liquid (e.g., water) and the surface itself is minimal. This interaction may be evaluated in terms of angle of contact of a drop of water deposited on the surface considered, measured as angle formed at the surface-liquid interface. A smaller angle of contact is due to the tendency of the drop to flatten out on the surface, and vice versa.

Figure 2A shows a drop of water L₁ arranged on a hydrophilic surface S₁. In this case, the drop L₁ shows an angle of contact θ = θ₁ with the surface S₁ of less than 90°. In general, a surface is considered hydrophilic if it presents characteristics of wettability such that, when a drop is deposited thereon, the angle of contact between the surface and the drop (angle θ) has a value of less than 90°, in particular equal to or less than approximately 40°.

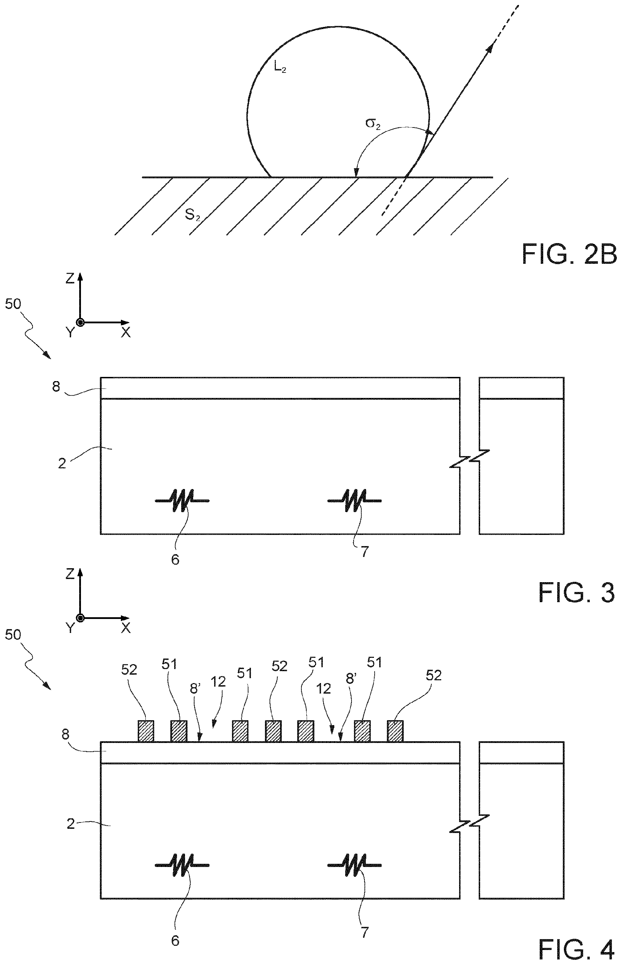

Figure 2B shows a drop of water L₂ arranged on a hydrophobic surface S₂. In this case, the angle of contact θ = θ₂ is greater than 90°. A surface is considered hydrophobic if it presents characteristics of wettability such that, when a drop is deposited thereon, the angle of contact between the surface and the drop (angle θ) has a value greater than 90°.

Therefore, in general, the hydrophobic layer 10 is of a material having hydrophobic characteristics, namely, such that a drop of liquid (e.g., water) deposited thereon presents an angle of contact θ greater than 90°. Preferably, the angle of contact is greater than 100°, for example 106°.

A manufacturing method of the chip 1 of Figure 1 is now described.

With reference to Figure 3, a wafer 50 is provided, for example of semiconductor material, in particular silicon, with a thickness, along Z, for example in the range of 200 to 800 µm. The wafer 50 comprises the substrate 2. The wafer 50 comprises the hydrophilic layer 8, for example formed by deposition of appropriate material (e.g., SiO₂, SiN, SiC, Si, hydrophilic metal, or more generally other hydrophilic materials compatible with the process, e.g., PCR, to be carried out using the chip 1), or thermal oxidation (forming, in this case, a silicon oxide layer).

The hydrophilic layer 8 has a thickness, along Z, for example in the range of 10 and 100 µm.

The wafer 50 is likewise machined to form the heaters 6 and the temperature sensors 7 in the case where these are of an integrated type. These machining steps are known and are not described in detail herein.

Then, Figure 4, the hydrophobic layer 10 is in part formed, in particular using the screen-printing technique.

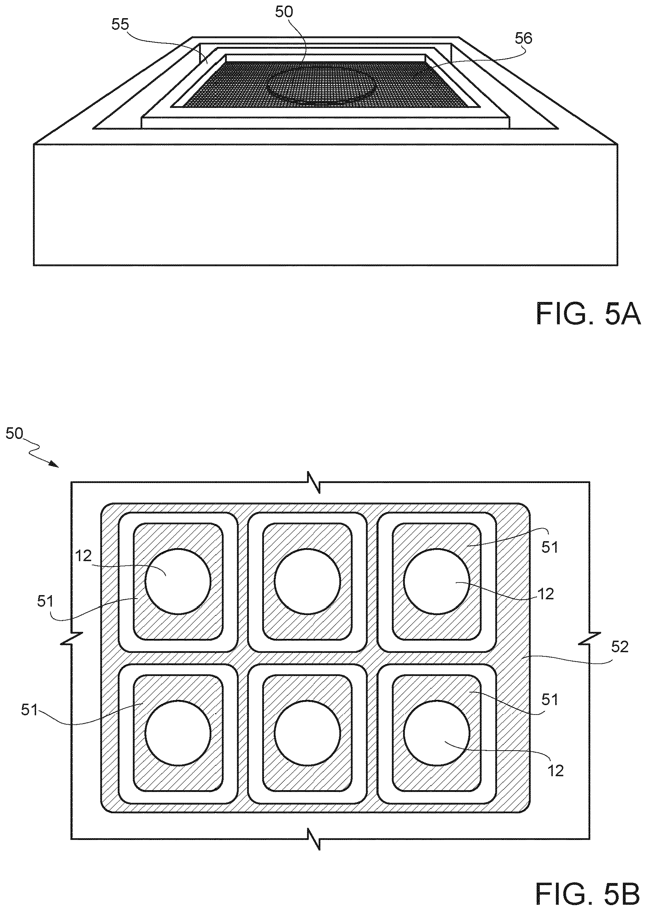

For this purpose, as illustrated in Figure 5A, the wafer 50 is arranged in a frame 55, provided with a lattice 56 defining the shape and dimensions of the hydrophobic layer 8 to be formed on the wafer 50.

As illustrated in Figure 5B (which shows a top view, in the plane XY, of the wafer 50), a paste layer is then "printed" on the wafer 50, in particular on the hydrophilic layer 8, to form first regions 51 and second regions 52 not connected together. The first regions 51 have, in this example, a quadrangular shape (other generally polygonal shapes are possible) and delimit internally the through openings 12. The second regions 52 form a "cobweb" that surrounds the first regions 51 at a distance. The second regions 52 have, in this process step, a thickness comprised between 50 and 150 µm.

As discussed previously, the choice of the material of the paste is made according to the desired characteristics of wettability and biocompatibility.

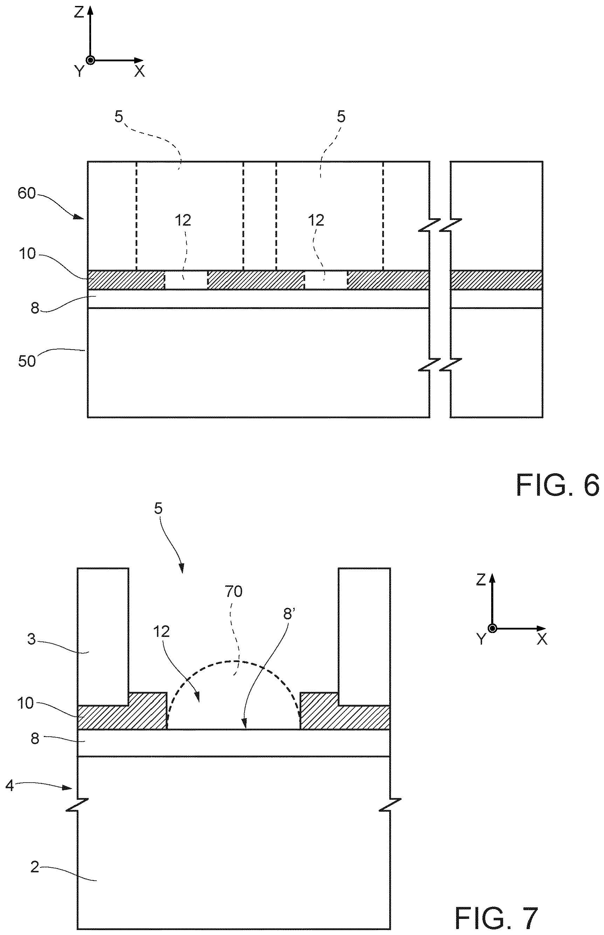

Then, Figure 6, a step of mechanical coupling between the wafer 50 and a further wafer 60 is carried out; the wafer 60 has the wells 5 previously described with reference to the first die 3. In this regard, using the pick and place technique, the wafer 60, which has the wells 5, is coupled to the wafer 50 at the hydrophobic layer 10. Alignment marks, or other solutions, are envisaged for aligning each well 5 correctly with a respective through opening 12. A mutual pressure is then exerted between the wafers 50, 60 such that the paste layer of the second regions 52 expands, reaching and joining the first regions 51 to form the completely connected hydrophobic layer 10 described previously.

A polymerization step is then carried out to enable solidification of the paste. This step varies according to the type of functionalization chemistry that is added to the paste used. For instance, acrylic, epoxy, or silicone pastes may be polymerized using UV rays and/or heat, or may not require a particular polymerization step, solidifying at environmental temperature and humidity in a pre-set time (e.g., a few hours) that is typically indicated by the manufacturer of the paste.

The steps previously described are carried out to form a plurality of chips 1 simultaneously. A step of dicing of the stack formed by the wafers 50 and 60 pasted together enables separation of the chips 1 from one another.

Figure 7 shows, in lateral sectional view, a portion of the chip 1 that includes a single reaction chamber, where it is appreciated that the hydrophobic layer 10 simultaneously carries out pasting of the dice 3, 4 and delimits (or defines) the region that is intended to house the solution to be analysed (here represented as a drop 70 with dashed line).

In use, drops of a fluid, or liquid, 70 that represent the sample to be analysed are selectively deposited at the through openings 12, in direct contact with the surface portions 8' of the hydrophilic layer 8 exposed through the hydrophobic layer 10. The liquid extends to cover completely or partially these surface portions 8' (having a good wettability and a small angle of contact), but does not cover portions of the hydrophobic layer 10 surrounding the respective surface portions 8'. In fact, as has been said, the hydrophobic layer 10 has a low wettability and a large angle of contact. In this way, there is a marked confinement of the drops of liquid within the through openings 12.

The chip 1 may find application in systems or devices for biological analysis (typically of a disposable type), for example PCR systems, or generic fluorescence-based diagnostic systems. For instance, according to an aspect of the present invention, one or more of the regions 8' (namely, the surface portions of the hydrophilic layer 8 exposed through the through openings 12) are functionalized through grafting of receptors or the like (in particular, receptor biomolecules).

The functionalization step may be carried out using an automated spotting technique of a per se known type, which basically envisages the use of a mechanical arm, which automatically picks up the biological material to be deposited (in liquid solution) and, with micrometric precision, deposits drops of this biological material selectively in the reaction chambers, to form detection regions. Typically, each of these drops is of a few picolitres, but the drops may have a volume of up to 1-5 µl or more according to the application and to the size of each reaction chamber.

The detection regions comprise, for example, a given type of receptors, such as biomolecules (DNA, RNA, proteins, antigens, antibodies, etc.) or micro-organisms or parts thereof (bacteria, viruses, spores, cells, organelles, etc.) or any chemical element used for detecting an analyte. The receptors, provided with specific markers, for example fluorescent markers, are grafted on the surface 8'. According to alternative embodiments, the receptors may be free in solution instead of being grafted to the device, according to the application for which the device is used. However, solid-phase assays are generally preferred since they enable washing of non-grafted material and therefore increase the sensitivity and simplicity of the detection assays.

The term "receptor" is here meant to indicate any member of a pair or multiple of elements that can bind together (the so-called binding pairs) so that the receptor will couple or react with, and thus detect, its binding mate (or mates) with which it can bind. Therefore, the receptors include traditional receptors, such as protein and ligand receptors, but also any member of a multiplicity of elements designed to interact or mate, such as lectins, carbohydrates, streptavidins, biotins, proteins, substrates, oligonucleotides, nucleic acids, porphyrins, metal ions, antibodies, antigens, and the like.

When these receptors are set in direct contact with a sample to be analysed, the presence in this sample of molecules capable of coupling or interacting with the receptor activates specific markers, for example fluorescent markers, which, when excited with light radiation of a certain wavelength λₑ, emit a light radiation thereof having a wavelength λ_{f} different from the wavelength λₑ. The markers are activated (namely, they emit a fluorescent radiation at a wavelength λ_{f}) only when the mate (or mates) with which the receptor can bind couples or interacts with the receptor.

The phenomenon of fluorescence is particularly useful in research and in diagnostic methods that envisage the use of devices obtained using MEMS technology.

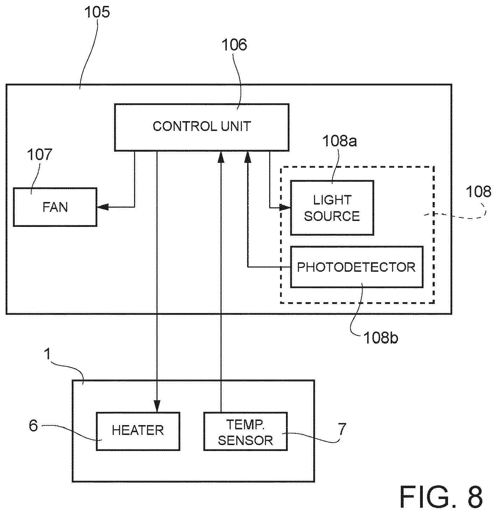

With reference to Figure 8, for the amplification of the nucleic material and for the recognition step it is possible to use the chip 1, manufactured as described with reference to Figures 3-6. In this example, the chip 1 comprises the heaters manufactured in integrated form on the back of the substrate 2, or coupled to the back of the substrate 2. Temperature sensors, which are also integrated on the back of the substrate 2 or coupled thereto, may moreover be present.

The chip 1 is loaded with drops of liquid that form the biological samples to be analysed and, then, is introduced into a thermocycler 105 for carrying out biochemical analysis. The thermocycler 105, known in the state of the art, is configured to receive one or more chips 1 mounted on purposely designed cards and comprises, in general, at least one control unit 106, a cooling device (e.g., a fan) 107, and a detection device 108 including a light source 108a for illuminating the sample to be analysed with a light radiation having a first wavelength and a photodetector 108b for acquiring a light radiation having a second wavelength, emitted by the sample in response to the illuminating light radiation.

The control unit 106 is connectable to the heaters and to the temperature sensors of the chip 1 through appropriate connectors and, exploiting the temperature sensors on board the chip 1 itself, controls the heaters and the cooling device 107 for carrying out pre-set thermal cycles.

Once the biochemical processes are finished, the detection device 108, which is typically of an optical type, verifies whether in the sample processed (namely, in the respective drop of liquid) given substances (for example, given nucleotide sequences) are present or absent. Optical detection typically exploits fluorophores, which, during processing of the sample, selectively bind to the substances to be recognised, emitting a characteristic light radiation. Other types of detection are, however, possible, according to the requirements and availability of the state of the art.

The advantages of the present invention emerge clearly from the preceding description.

In particular, according to the present invention it is possible to control the characteristics of wettability of surfaces with very high precision given by the precision afforded by the screen-printing technique used.

Furthermore, the manufacturing steps are considerably simplified in so far as no further lithographic and etching steps are required for definition of the hydrophobic layer.

Furthermore, the embodiment of the hydrophobic layer 10 described, at the same time, guarantees thermal resistance, control of thickness, high patterning resolution and chemical inertia.

Modifications and variations may be made to what has been described and illustrated previously, without thereby departing from the scope of the present invention, as defined in the annexed claims.

## Claims

1. A chip (1) for biochemical reactions comprising:
- a first body (3) including a plurality of first through openings (5) arranged according to an arrangement pattern;
- a second body (4), having a hydrophilic surface (8), coupled to the first body (3) on the hydrophilic surface (8) ;
- an intermediate layer (10), which extends over the hydrophilic surface (8) and forms a coupling interface between the first and the second bodies,
**characterised in that** the intermediate layer (10) is of hydrophobic material and has a plurality of second through openings (12), which are arranged according to said arrangement pattern and through which respective regions (8') of the hydrophilic surface (8) are exposed,
wherein the first and the second bodies (3, 4) are coupled together so that a respective second through opening (12) and a portion of the intermediate layer (10) surrounding said respective second through opening (12) are exposed through each of said first through openings (5).

2. The chip according to claim 1, wherein the intermediate layer (10) extends continuously in direct contact with the hydrophilic surface.

3. The chip according to claim 1 or claim 2, wherein the second body (4) comprises a substrate (2) of semiconductor material and a layer of hydrophilic material (8) on the substrate, said layer of hydrophilic material (8) forming said hydrophilic surface of the second body (4).

4. The chip according to claim 3, wherein the substrate is of silicon, and the layer of hydrophilic material (8) is of a material chosen from among: silicon oxide, silicon nitride, silicon carbide, silicon, hydrophilic metal.

5. The chip according to any one of the preceding claims, wherein the intermediate layer (10) has a viscosity in the range 10 to 80 Pa per second, and characteristics of wettability determined by an angle of contact of at least 40° greater than that of the hydrophilic layer.

6. The chip according to any one of the preceding claims, wherein the intermediate layer (10) is of a silicone paste.

7. The chip according to any one of the preceding claims, wherein the intermediate layer (10) is of a material chosen from among: silicone, epoxy or acrylic pastes.

8. The chip according to any one of the preceding claims, wherein the second through openings (12) have an oval, or a circular or a polygonal shape having a first diameter, and wherein the first through openings (5) have an oval, or a circular or a polygonal shape having a second diameter, the second diameter being greater than the first diameter.

9. The chip according to any one of the preceding claims, wherein the first body (3) is of hydrophobic material.

10. The chip according to any one of the preceding claims, wherein one or more of said second through openings (12) houses at least one detection region comprising one or more receptors designed to establish a bond with respective one or more binding mates.

11. A system for biological analysis, including at least one chip (1) according to any one of claims 1-10.

12. A manufacturing method of a chip (1) for biochemical reactions, comprising the steps of:
- providing a first body (3) including a plurality of first through openings (5) arranged according to an arrangement pattern;
- providing a second body (4), having a hydrophilic surface (8);
- forming an intermediate layer (10) on the hydrophilic surface (8); and
- coupling together the first body and the second body by the intermediate layer (10),
**characterised in that** the step of forming the intermediate layer (10) comprises depositing hydrophobic material using the screen-printing technique, forming a plurality of second through openings (12) through said hydrophobic material, which are arranged according to said arrangement pattern and through which respective regions (8') of the hydrophilic surface (8) are exposed,
wherein the step of coupling together the first and the second bodies includes aligning each of said first through openings (5) to a respective second through opening (12).

13. The method according to claim 12, wherein the second through openings (12) have an oval, or a circular or a polygonal shape having a first diameter, and wherein the first through openings (5) have an oval, or a circular or a polygonal shape having a second diameter, the second diameter being greater than the first diameter so that through the first through openings (5) respective first through openings (5) and a portion of the intermediate layer (10) surrounding said respective second through opening (12) are exposed.

14. The method according to claim 12 or claim 13, wherein the step of coupling together the first and the second bodies (3, 4) includes pressing the first body on the second body.

15. The method according to any one of claims 12-14, wherein said hydrophobic material is a silicone paste.

16. The method according to any one of claims 12-14, wherein said hydrophobic material is chosen from one of the following: silicone, epoxy or acrylic pastes.

17. The method according to any one of claims 12-14, wherein said hydrophobic material has a viscosity in the range 10 to 80 Pa per second, and characteristics of wettability determined by an angle of contact of at least 40° greater than that of the hydrophilic layer.

18. The method according to any one of claims 12-14, comprising the step of functionalizing said region (8') of the hydrophilic surface (8) to form at least one detection region comprising one or more receptors designed to establish a bond with respective one or more binding mates.
